# EUROPEAN PATENT SPECIFICATION

(11) **EP 3 265 489 B1**
(45) Date of publication and mention of the grant of the patent: **08.05.2019**
(21) Application number: 16708978.8
(22) Date of filing: 04.03.2016
(51) Int. Cl.: A61K 39/00, A61K 38/37, A61K 39/395, A61K 9/08, C07K 14/755, C07K 16/28

(54) **COMPOUNDS FOR IMPROVING THE HALF-LIFE OF VON WILLEBRAND FACTOR**
VERBINDUNGEN ZUR ERHÖHUNG DER HALBWERTSZEIT DES VON-WILLEBRAND-FAKTORS
COMPOSÉS POUR AMÉLIORER LA DURÉE DE LA DEMI-VIE DU FACTEUR VON WILLEBRAND

(30) Priority: 06.03.2015 EP 15158088
(43) Date of publication of application: 10.01.2018
(73) Proprietor: CSL Behring Lengnau AG, 2543 Lengnau BE (CH)
(72) Inventor: MOSES, Michael, 61279 Graevenwiesbach (DE); SCHULTE, Stefan, 35043 Marburg (DE); DICKNEITE, Gerhard, 35043 Marburg (DE); KALINA, Uwe, 35041 Marburg (DE); PESTEL, Sabine, 35041 Marburg (DE)
(74) Representative: Ludwig, Holger
(86) International application number: PCT/EP2016/054650
(87) International publication number: WO 2016/142289

(56) References cited:
- WO-A1-2013/167303
- Y. SANO ET AL: "Properties of Blocking and Non-blocking Monoclonal Antibodies Specific for Human Macrophage Galactose-type C-type Lectin (MGL/ClecSF10A/CD301)", JOURNAL OF BIOCHEMISTRY, vol. 141, no. 1, 13 December 2006 (2006-12-13), pages 127-136, XP055201902, ISSN: 0021-924X, DOI: 10.1093/jb/mvm017

### SEQUENCE LISTING

<110> CSL Behring Recombinant Facility AG
<120> Compounds for improving the Half-life of Von Willebrand Factor
<130> A242
<150> EP2015158088.3
   <151> 2015-03-06
<160> 2
<170> PatentIn version 3.5
<210> 1
   <211> 316
   <212> PRT
   <213> Homo sapiens
<220>
   <221> MISC_FEATURE
   <222> (35)..(35)
   <223> Xaa is Cys or Arg
<220>
   <221> MISC_FEATURE
   <222> (73)..(73)
   <223> Xaa is Arg or Lys
<220>
   <221> MISC_FEATURE
   <222> (100)..(100)
   <223> Xaa is Thr or Met
<220>
   <221> MISC_FEATURE
   <222> (203)..(203)
   <223> Xaa is Ala or Gly
<400> 1
   Met Thr Arg Thr Tyr Glu Asn Phe Gln Tyr Leu Glu Asn Lys Val Lys
   1               5                   10                  15
   Val Gln Gly Phe Lys Asn Gly Pro Leu Pro Leu Gln Ser Leu Leu Gln
               20                  25                  30
   Arg Leu Xaa Ser Gly Pro Cys His Leu Leu Leu Ser Leu Gly Leu Gly
           35                  40                  45
   Leu Leu Leu Leu Val Ile Ile Cys Val Val Gly Phe Gln Asn Ser Lys
       50                  55                  60
   Phe Gln Arg Asp Leu Val Thr Leu Xaa Thr Asp Phe Ser Asn Phe Thr
   65                  70                  75                  80
   Ser Asn Thr Val Ala Glu Ile Gln Ala Leu Thr Ser Gln Gly Ser Ser
   85 90 95
   Leu Glu Glu Xaa Ile Ala Ser Leu Lys Ala Glu Val Glu Gly Phe Lys
   100 105 110
   Gln Glu Arg Gln Ala Gly Val Ser Glu Leu Gln Glu His Thr Thr Gln
   115 120 125
   Lys Ala His Leu Gly His Cys Pro His Cys Pro Ser Val Cys Val Pro
   130 135 140
   Val His Ser Glu Met Leu Leu Arg Val Gln Gln Leu Val Gln Asp Leu
   145 150 155 160
   Lys Lys Leu Thr Cys Gln Val Ala Thr Leu Asn Asn Asn Ala Ser Thr
   165 170 175
   Glu Gly Thr Cys Cys Pro Val Asn Trp Val Glu His Gln Asp Ser Cys
   180 185 190
   Tyr Trp Phe Ser His Ser Gly Met Ser Trp Xaa Glu Ala Glu Lys Tyr
   195 200 205
   Cys Gln Leu Lys Asn Ala His Leu Val Val Ile Asn Ser Arg Glu Glu
   210 215 220
   Gln Asn Phe Val Gln Lys Tyr Leu Gly Ser Ala Tyr Thr Trp Met Gly
   225 230 235 240
   Leu Ser Asp Pro Glu Gly Ala Trp Lys Trp Val Asp Gly Thr Asp Tyr
   245 250 255
   Ala Thr Gly Phe Gln Asn Trp Lys Pro Gly Gln Pro Asp Asp Trp Gln
   260 265 270
   Gly His Gly Leu Gly Gly Gly Glu Asp Cys Ala His Phe His Pro Asp
   275 280 285
   Gly Arg Trp Asn Asp Asp Val Cys Gln Arg Pro Tyr His Trp Val Cys
   290 295 300
   Glu Ala Gly Leu Gly Gln Thr Ser Gln Glu Ser His
   305 310 315
<210> 2
   <211> 316
   <212> PRT
   <213> Homo sapiens
<400> 2
   Met Thr Arg Thr Tyr Glu Asn Phe Gln Tyr Leu Glu Asn Lys Val Lys
   1               5                   10                  15
   Val Gln Gly Phe Lys Asn Gly Pro Leu Pro Leu Gln Ser Leu Leu Gln
               20                  25                  30
   Arg Leu Cys Ser Gly Pro Cys His Leu Leu Leu Ser Leu Gly Leu Gly
           35                  40                  45
   Leu Leu Leu Leu Val Ile Ile Cys Val Val Gly Phe Gln Asn Ser Lys
       50                  55                  60
   Phe Gln Arg Asp Leu Val Thr Leu Arg Thr Asp Phe Ser Asn Phe Thr
   65                  70                  75                  80
   Ser Asn Thr Val Ala Glu Ile Gln Ala Leu Thr Ser Gln Gly Ser Ser
                   85                  90                  95
   Leu Glu Glu Thr Ile Ala Ser Leu Lys Ala Glu Val Glu Gly Phe Lys
               100                 105                 110
   Gln Glu Arg Gln Ala Gly Val Ser Glu Leu Gln Glu His Thr Thr Gln
           115                 120                 125
   Lys Ala His Leu Gly His Cys Pro His Cys Pro Ser Val Cys Val Pro
       130                 135                 140
   Val His Ser Glu Met Leu Leu Arg Val Gln Gln Leu Val Gln Asp Leu
   145                 150                 155                 160
   Lys Lys Leu Thr Cys Gln Val Ala Thr Leu Asn Asn Asn Ala Ser Thr
                   165                 170                 175
   Glu Gly Thr Cys Cys Pro Val Asn Trp Val Glu His Gln Asp Ser Cys
               180                 185                 190
   Tyr Trp Phe Ser His Ser Gly Met Ser Trp Ala Glu Ala Glu Lys Tyr
           195                 200                 205
   Cys Gln Leu Lys Asn Ala His Leu Val Val Ile Asn Ser Arg Glu Glu
       210                 215                 220
   Gln Asn Phe Val Gln Lys Tyr Leu Gly Ser Ala Tyr Thr Trp Met Gly
   225                 230                 235                 240
   Leu Ser Asp Pro Glu Gly Ala Trp Lys Trp Val Asp Gly Thr Asp Tyr
                   245                 250                 255
   Ala Thr Gly Phe Gln Asn Trp Lys Pro Gly Gln Pro Asp Asp Trp Gln
               260                 265                 270
   Gly His Gly Leu Gly Gly Gly Glu Asp Cys Ala His Phe His Pro Asp
           275                 280                 285
   Gly Arg Trp Asn Asp Asp Val Cys Gln Arg Pro Tyr His Trp Val Cys
       290                 295                 300
   Glu Ala Gly Leu Gly Gln Thr Ser Gln Glu Ser His
   305                 310                 315

## Description

### FIELD OF THE INVENTION

The present invention relates to products and methods for improving treatment of blood coagulation disorders.

### BACKGROUND OF THE INVENTION

There are various bleeding disorders caused by deficiencies of blood coagulation factors. The most common disorders are hemophilia A and B, resulting from deficiencies of blood coagulation factor VIII (FVIII) and IX, respectively. Another known bleeding disorder is von Willebrand's disease (VWD).

In plasma FVIII exists mostly as a noncovalent complex with von Willebrand factor (VWF), and its coagulant function is to accelerate factor IXa dependent conversion of factor X to Xa.

Classic hemophilia or hemophilia A is an inherited bleeding disorder. It results from a chromosome X-linked deficiency of blood coagulation FVIII, and affects almost exclusively males with an incidence of between one and two individuals per 10,000. The X-chromosome defect is transmitted by female carriers who are not themselves hemophiliacs. The clinical manifestation of hemophilia A is an increased bleeding tendency.

In severe hemophilia A patients undergoing prophylactic treatment FVIII has to be administered intravenously (i.v.) about 3 times per week due to the short plasma half-life of FVIII of about 12 to 14 hours. Each i.v. administration is cumbersome, associated with pain and entails the risk of an infection especially as this is mostly done at home by the patients themselves or by the parents of children being diagnosed for hemophilia A.

It would thus be highly desirable to increase the half-life of FVIII so that pharmaceutical compositions containing FVIII have to be administered less frequently.

Several attempts have been made to prolong the half-life of non-activated FVIII either by reducing its interaction with cellular receptors (WO 03/093313 A2, WO 02/060951 A2), by covalently attaching polymers to FVIII (WO 94/15625, WO 97/11957 and US 4970300), by encapsulation of FVIII (WO 99/55306), by introduction of novel metal binding sites (WO 97/03193), by covalently attaching the A2 domain to the A3 domain either by peptidic (WO 97/40145 and WO 03/087355) or disulfide linkage (WO 02/103024A2) or by covalently attaching the A1 domain to the A2 domain (WO2006/108590).

Another approach to enhance the functional half-life of FVIII or VWF is by PEGylation of FVIII (WO 2007/126808, WO 2006/053299, WO 2004/075923) or by PEGylation of VWF (WO 2006/071801) which pegylated VWF by having an increased half-life would indirectly also enhance the half-life of FVIII present in plasma. Also fusion proteins of FVIII have been described (WO 2004/101740, WO2008/077616 and WO 2009/156137).

WO 2013/167303 describes a further method for increasing the half-life of VWF using sugar residues derived from ABO(H) blood group antigen.

VWF, which is missing, functionally defect or only available in reduced quantity in different forms of von Willebrand disease (VWD), is a multimeric adhesive glycoprotein present in the plasma of mammals, which has multiple physiological functions. During primary hemostasis VWF acts as a mediator between specific receptors on the platelet surface and components of the extracellular matrix such as collagen. Moreover, VWF serves as a carrier and stabilizing protein for procoagulant FVIII. VWF is synthesized in endothelial cells and megakaryocytes as a 2813 amino acid precursor molecule. The amino acid sequence and the cDNA sequence of wild-type VWF are disclosed in Collins et al. 1987, Proc Natl. Acad. Sci. USA 84:4393-4397. The precursor polypeptide, pre-pro-VWF, consists of a 22-residue signal peptide, a 741- residue pro-peptide and the 2050-residue polypeptide found in mature plasma VWF (Fischer et al., FEBS Lett. 351: 345-348, 1994). After cleavage of the signal peptide in the endoplasmatic reticulum a C-terminal disulfide bridge is formed between two monomers of VWF. During further transport through the secretory pathway N-linked and O-linked carbohydrate side chains are added. More importantly, VWF dimers are multimerized via N-terminal disulfide bridges and the propeptide of 741 amino acids length is cleaved off by the enzyme PACE/furin in the late Golgi apparatus. The propeptide as well as the high-molecular-weight multimers of VWF (VWF-HMWM) are stored in the Weibel-Palade bodies of endothelial cells or in the α-granules of platelets.

Once secreted into plasma the protease ADAMTS13 cleaves VWF within the A1 domain of VWF. Plasma VWF therefore consists of a whole range of multimers ranging from single dimers of 500 kDa to multimers consisting of up to more than 20 dimers of a molecular weight of over 10,000 kDa. The VWF-high molecular weight multimers (HMWM) have the strongest hemostatic activity, which can be measured in ristocetin cofactor activity (VWF:RCo). The higher the ratio of VWF:RCo/VWF antigen, the higher the relative amount of high molecular weight multimers.

Defects in VWF are causal to von Willebrand disease (VWD), which is characterized by a more or less pronounced bleeding phenotype. VWD type 3 is the most severe form in which VWF is completely missing, VWD type 1 relates to a quantitative loss of VWF and its phenotype can be very mild. VWD type 2 relates to qualitative defects of VWF and can be as severe as VWD type 3. VWD type 2 has many sub forms some of them being associated with the loss or the decrease of high molecular weight multimers. Von VWD type 2a is characterized by a loss of both intermediate and large multimers. VWD type 2B is characterized by a loss of highest-molecular-weight multimers.

VWD is the most frequent inherited bleeding disorder in humans and can be treated by replacement therapy with concentrates containing VWF of plasmatic or recombinant origin.

In plasma FVIII binds with high affinity to VWF, which protects it from premature catabolism and thus, plays in addition to its role in primary hemostasis a crucial role to regulate plasma levels of FVIII and as a consequence is also a central factor to control secondary hemostasis. The half-life of non-activated FVIII bound to VWF is about 12 to 14 hours in plasma. In von Willebrand disease type 3, where no or almost no VWF is present, the half-life of FVIII is only about 6 hours, leading to symptoms of mild to moderate hemophilia A in such patients due to decreased concentrations of FVIII. The stabilizing effect of VWF on FVIII has also been used to aid recombinant expression of FVIII in CHO cells (Kaufman et al. 1989, Mol Cell Biol).

There is a need for products and methods for increasing the half-life of VWF, FVIII or both factors.

### SUMMARY OF THE INVENTION

The inventors of this application found that VWF monomers strongly bind to calcium-type lectin domain family 10 member A (CLEC10A), a receptor protein present on macrophages. In particular, it was found that an antibody capable of binding to the mouse ortholog of CLEC10A had an inhibiting effect on the clearance of VWF in mice. Thus, by reducing the clearance of VWF, inhibitory antibodies capable of binding to CLEC10A prolong the half-life of VWF in plasma. By administering such inhibitory antibodies the half-life of FVIII can also be increased.

The present disclosure therefore relates to the subject matter defined in items [1] to [18]:
[1] A compound capable of binding to the human receptor protein CLEC10A or an ortholog thereof for use in the treatment of a blood coagulation disorder.
[2] The compound for use according to item [1], wherein said compound is capable of inhibiting the binding of von Willebrand factor to CLEC10A.
[3] The compound for use according to item [1] or [2], wherein said compound binds specifically to the CLEC10A.
[4] The compound for use according to any one of the preceding items, wherein said compound is an antibody or a fragment thereof.
[5] The compound for use according to item [4], wherein said antibody is a monoclonal antibody.
[6] The compound for use according to any one of the preceding items, wherein said CLEC10A has the amino acid sequence shown in SEQ ID NO: 1 or 2.
[7] The compound for use according to any one of the preceding items, wherein the half-life of von Willebrand factor is increased by the treatment.
[8] The compound for use according to any one of the preceding items, wherein the half-life of Factor VIII is increased by the treatment.
[9] The compound for use according to any one of the preceding items, wherein said treatment further comprises administering a polypeptide selected from the group consisting of Factor VIII, von Willebrand factor and combinations thereof.
[10] The compound for use according to item [9], wherein said compound and said polypeptide are administered separately.
[11] The compound for use according to any one of the preceding items, wherein said blood coagulation disorder is hemophilia A or von Willebrand disease.
[12] A pharmaceutical kit comprising (i) a first compound as defined in any one of items 1 to 6 and (ii) a polypeptide selected from the group consisting of Factor VIII, von Willebrand factor and combinations thereof.
[13] The pharmaceutical kit of item [12], wherein said compound and said polypeptide are contained in separate compositions.
[14] The use of a compound as defined in any one of items [1] to [6] for increasing the half-life or reducing the clearance of von Willebrand Factor.
[15] The use of a compound as defined in any one of items [1] to [6] for increasing the half-life of Factor VIII.
[16] A compound as defined in any one of items [1] to [6] for use in prolonging the half-life of von Willebrand factor in a therapeutic treatment.
[17] A method of increasing the half-life or reducing the clearance of von Willebrand Factor *in vivo,* comprising administering to a subject an effective amount of a compound as defined in any one of items [1] to [6].
[18] A method of treating a blood coagulation disorder, comprising administering to a patient in need thereof an effective amount of a compound as defined in any one of items [1] to [6].

### DESCRIPTION OF THE DRAWINGS

**Figure 1****: Interaction of monomeric human VWF with recombinant human CLEC10A** (see Example 1).
**Figures 2** and **3****: Interaction of monomeric human VWF with both recombinant human CLEC10A and the CLEC10A orthologous mouse proteins (MGL1 and MGL2)** (see Example 2).
**Figure 4****: Inhibition of VWF-binding to MGL2 in the presence of a neutralizing anti-MGL1/2 antibody** (see Example 3).
**Figure 5****: PK of human VWF in the presence of an anti-MGL1/2 antibody neutralizing the respective receptor function *in vivo*** (see Example 4).

VWF-deficient mice received 8 mg per kg body weight of a polyclonal goat anti-MGL1/2 antibody. A nonspecific goat antibody was used as control treatment. Subsequently, human VWF (200 IU/kg body weight) was injected and VWF:Ag is presented as the percentage of the injected VWF dose recovered in plasma at the indicated time after injection. The administration of the inhibitory anti-MGL1/2 antibody led to a decrease in VWF clearance, when compared with the group receiving the control antibody.

### DETAILED DESCRIPTION

In a first aspect, the present invention pertains to an antibody capable of binding to the human receptor protein CLEC10A or an ortholog thereof for use in the treatment of a blood coagulation disorder.

In other aspects, the present invention pertains to a compound capable of binding to human CLEC10A or an ortholog thereof for use in the treatment of a blood coagulation disorder, wherein said compound
(i) is an antibody,
(ii) is capable of specifically binding to human CLEC10A or an ortholog thereof,
(iii) does not comprise an accessible sugar residue that is part of or derived from ABO(H) blood group antigens,
(iv) does not bind to the receptor protein CLEC10A or an ortholog thereof via a carbohydrate structure that may be part of said compound,
(v) does not bind to the human ASGP receptor,
(vi) does not bind to the human receptor CLEC4M,
(vii) does not bind to the human receptor SCARA5,
(viii) does not bind to the human receptor MMR,
(ix) does not bind to the human receptor CLEC4F, and/or
(x) does not bind to the human receptor COLEC12.

### CLEC10A

CLEC10A, also known as macrophage Gal-type lectin, is a human type II transmembrane receptor protein of the CLEC family. Further synonyms are C-type lectin superfamily member 14, Macrophage lectin 2, and CD301. CLEC10A is closely related to the hepatic ASGPR proteins but is expressed by intermediate monocytes, macrophages and dendritic cells. Nevertheless, CLEC10A and ASGPR are different proteins with distinct cellular localization and carbohydrate specificity. CLEC10A was reported to be involved in the recognition of pathogens by dendritic cells and to selectively recognize tumor-associated glycoproteins in cancer incidence (van Vliet et al. (2005) International Immunology, 17: 661-669; Napoletano et al. (2012) European Journal of Immunology, 42: 936-945). This receptor was described as mediating binding to glycoproteins that contain terminal α- and β-linked GalNAc residues. O-linked carbohydrate structures, such as the Tn-antigen (GalNAc α-linked to serine/threonine) and sialyl-Tn-antigen structures, have been identified as preferred interaction partners of CLEC10A (van Vliet et al. (2005) International Immunology, 17: 661-669; Saeland et al. (2007) Cancer Immunology, Immunotherapy, 56: 1225-1236; van Vliet et al. (2008) International Immunology, 29: 83-90; Denda-Nagai et al. (2010) The Journal of Biological Chemistry, 285: 19193-19204; Mortezai et al. (2013) Glycobiology, 23: 844-852). Many tumor cells display aberrant glycosylation due to altered expression levels and activities of glycosyltransferases, which results in abnormal expression of glycans, such as Tn antigen (van Vliet et al. (2008) International Immunology, 29: 83-90.).

As used herein, the term "CLEC10A" refers to a human protein having or consisting of the amino acid sequence as shown in SEQ ID NO:1 or a naturally occurring variant thereof. CLEC10A includes, but is not limited to, proteins having or consisting of the amino acid sequences as shown in the UniProt database under identifiers Q8IUN9-1, Q8IUN9-2, and Q8IUN9-3. Most preferably, the CLEC10A comprises or consists of the amino acid sequence as shown in SEQ ID NO:2.

Orthologs of CLEC10A have been identified in several species, including mouse, rat and zebrafish. Humans carry a single gene for CLEC10A, while mouse has two closely related MGL1 and MGL2 genes. The murine receptor proteins are also expressed on macrophages and immature dendritic cells. Human CLEC10A and the murine receptor proteins show a high degree of homology on amino acid level. Within the carbohydrate recognition domain, human CLEC10A shares around 60% amino acid sequence identity with both mouse MGL1 and mouse MGL2 (Suzuki et al. (1996) The Journal of Immunology, 156: 128-135). Similar ligand preference is exhibited by human CLEC10A, mouse MGL1 and mouse MGL2, and previously reported binding studies have demonstrated that all three receptor proteins recognize Gal- and GalNAc-related (Suzuki et al. (1996) The Journal of Immunology, 156: 128-135; Oo-puthinan et al. (2008) Biochimica et Biophysica Acta, 1780: 89-100). MGL1 and MGL2 are highly homologous to each other in their amino acid sequences (around 90% amino acid identity) and share a high degree of identity in the carbohydrate recognition domain (Tsuiji et al. (2002) The Journal of Biological Chemistry, 277: 28892-28901; Oo-puthinan et al. (2008) Biochimica et Biophysica Acta, 1780: 89-100).

Sano et. al 2006 disclose blocking and non-blocking monoclonal antibodies against CLEC10A but does not concern vWF binding.

Preferred orthologs in accordance with this disclosure include:
- orthologs from mouse (*mus musculus*) e.g. a polypeptide having or consisting of an amino acid sequence defined by one of UniProt identifiers P49300, F8WHB7 and Q8JZN1;
- ortholog(s) from rat (*rattus norvegicus*),) e.g. a polypeptide having or consisting of the amino acid sequence defined by UniProt identifier:P49301);
- ortholog(s) from rabbit (*oryctolagus cuniculus*),
- ortholog(s) from guinea pig (*cavia porcellus*),
- ortholog(s) from macaca *fascicularis* and
- ortholog(s) from macaca *mulatta.*

### Compound capable of binding to CLEC10A

The type or class of the compound capable of binding to CLEC10A (hereinafter referred to as "the compound") is not particularly limited. Preferably, however, the compound is a peptide or polypeptide, most preferably the compound is an antibody or a fragment thereof.

The term "antibody", as used herein, refers to an immunoglobulin molecule that binds to, or is immunologically reactive with, a particular antigen, and includes polyclonal, monoclonal, genetically engineered and otherwise modified forms of antibodies, including but not limited to chimeric antibodies, humanized antibodies, human antibodies, heteroconjugate antibodies (e.g., bispecific antibodies, diabodies, triabodies, and tetrabodies), single-domain antibodies (nanobodies) and antigen binding fragments of antibodies, including e.g., Fab', F(ab')2, Fab, Fv, rlgG, and scFv fragments. Moreover, unless otherwise indicated, the term "monoclonal antibody" (mAb) is meant to include both intact molecules, as well as, antibody fragments (such as, for example, Fab and F(ab')2 fragments) which are capable of binding to a protein. Fab and F(ab')2 fragments lack the Fc fragment of intact antibody, clear more rapidly from the circulation of the animal or plant, and may have less non-specific tissue binding than an intact antibody (Wahl et al, 1983, J. Nucl. Med. 24:316).

The antibody of the invention is capable of binding to at least one variant of CLEC10A. In a preferred embodiment, the antibody is capable of binding to the protein consisting of the amino acid sequence as shown in SEQ ID NO:1. In the most preferred embodiment, the antibody is capable of binding to the protein consisting of the amino acid sequence as shown in SEQ ID NO:2.

In other embodiments, the antibody is capable of binding to the extracellular domain of CLEC10A, e.g. to an epitope within amino acids 61 - 316 of SEQ ID NO:2.

Preferably, the antibody of the invention binds to the lectin binding site of CLEC10A.

Preferably, the antibody of the invention binds to CLEC10A via amino acid residues in the variable region. More preferably, the antibody of the invention does not contain an accessible sugar residue that is derived from ABO(H) blood group antigen, the antibody does not comprise an accessible sugar residue that is galactose, fucose or N-acetylgalactosamine. Even more preferably, the antibody does not contain a glycosylation site in the Fc region, e.g. the antibody is an IgG with a mutation of residue N297 according to the numbering of Kabat. Most preferably, the antibody is not glycosylated.

It is also preferred that the antibody specifically binds to CLEC10A. In one embodiment, the antibody is capable of binding to CLEC10A, but is not capable of binding to two or more, preferably to all of the following receptors: ASGPR1, COLEC12, CLEC4F, CLEC4M, SCARA5 and MMR.

In another embodiment, the antibody is capable of binding to CLEC10A, but is not capable of binding to ASGPR1 (UniProt identifier: P07306).

In another embodiment, the antibody is capable of binding to CLEC10A, but is not capable of binding to COLEC12 (UniProt identifier: Q5KU26).

In another embodiment, the antibody is capable of binding to CLEC10A, but is not capable of binding to CLEC4F (UniProt identifier: Q8N1N0).

In another embodiment, the antibody is capable of binding to CLEC10A, but is not capable of binding to CLEC4M (UniProt identifier: Q9H2X3).

In another embodiment, the antibody is capable of binding to CLEC10A, but is not capable of binding to SCARA5 (UniProt identifier: Q6ZMJ2).

In another embodiment, the antibody is capable of binding to CLEC10A, but is not capable of binding to MMR (UniProt identifier: P22897).

In yet another embodiment, the antibody is capable of binding to CLEC10A, but is not capable of binding to any one of the following receptors: ASGPR1, COLEC12, CLEC4F, CLEC4M, SCARA5 and MMR.

In another embodiment, the antibody is capable of binding to at least one murine ortholog of CLEC10A. In that embodiment, the antibody may be capable of binding to MGL1, to MGL2, or to both MGL1 and MGL2. The antibody may be capable of binding to a protein having or consisting of the amino acid sequence defined in UniProt identifier No. P49300. The antibody may be capable of binding to a protein having or consisting of the amino acid sequence defined in UniProt identifier No. F8WHB7. The antibody may be capable of binding to a protein having or consisting of the amino acid sequence defined in UniProt identifier No. Q8JZN1.

In another embodiment, the antibody is capable of binding to the rat ortholog of CLEC10A. In another embodiment, the antibody is capable of binding to the rabbit ortholog of CLEC10A. In another embodiment, the antibody is capable of binding to the macaca *fascicularis* ortholog and/or to the macaca *mulatta* ortholog of CLEC10A.

The binding of the antibody to CLEC10A can be determined as described in Example 1 hereinbelow.

The dissociation constant K_{D} for the complex formed by CLEC10A and antibody is preferably less than 100 nM, more preferably less than 10 nM, most preferably less than 5 nM. Typically the K_{D} ranges from about 10 pM to about 100 nM, or from about 100 pM to about 10 nM, or from about 500 pM to about 5 nM.

Preferably, the antibody of this invention is a monoclonal antibody. The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced. Monoclonal antibodies can be prepared using a wide variety of techniques known in the art including the use of hybridoma, recombinant, and phage display technologies, or a combination thereof. (Harlow and Lane, "Antibodies, A Laboratory Manual" CSH Press 1988, Cold Spring Harbor N.Y.).

In other embodiments, including in vivo use of the anti-CLEC10A antibodies in humans, chimeric, primatized, humanized, or human antibodies can be used. In a preferred embodiment, the antibody is a human antibody or a humanized antibody, more preferably a monoclonal human antibody or a monoclonal humanized antibody.

The term "chimeric" antibody as used herein refers to an antibody having variable sequences derived from a non-human immunoglobulins, such as rat or mouse antibody, and human immunoglobulins constant regions, typically chosen from a human immunoglobulin template. Methods for producing chimeric antibodies are known in the art. See, e.g., Morrison, 1985, Science 229 (4719): 1202-7; Oi et al, 1986, BioTechniques 4:214-221; Gillies et al., 1985, J. Immunol. Methods 125: 191-202; U.S. Pat. Nos. 5,807,715; 4,816,567; and 4,816397.

"Humanized" forms of non-human (e.g., murine) antibodies are chimeric immunoglobulins, immunoglobulin chains or fragments thereof (such as Fv, Fab, Fab', F(ab')2 or other target-binding subsequences of antibodies) which contain minimal sequences derived from non-human immunoglobulin. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody can also comprise at least a portion of an immunoglobulin constant region (Fc), typically that of a human immunoglobulin template chosen. Humanization is a technique for making a chimeric antibody in which one or more amino acids or portions of the human variable domain have been substituted by the corresponding sequence from a non-human species. Humanized antibodies are antibody molecules generated in a non-human species that bind the desired antigen having one or more complementarity determining regions (CDRs) from the non-human species and framework (FR) regions from a human immunoglobulin molecule. Often, framework residues in the human framework regions will be substituted with the corresponding residue from the CDR donor antibody to alter, preferably improve, antigen binding. These framework substitutions are identified by methods well known in the art, e.g., by modeling of the interactions of the CDR and framework residues to identify framework residues important for antigen binding and sequence comparison to identify unusual framework residues at particular positions. See, e.g., Riechmann et al., 1988, Nature 332:323-7 and Queen et al, U.S. Patent Nos: 5,530,101; 5,585,089; 5,693,761; 5,693,762; and 6,180,370.

Antibodies can be humanized using a variety of techniques known in the art including, for example, CDR-grafting (EP239400; PCT publication WO 91/09967; U.S. Patent Nos. 5,225,539; 5,530,101 and 5,585,089), veneering or resurfacing (EP592106; EP519596; Padlan, 1991, Mol. Immunol, 28:489-498; Studnicka et al, 1994, Prot. Eng. 7:805-814; Roguska et al, 1994, Proc. Natl. Acad. Sci. 91:969-973, and chain shuffling (U.S. Patent No. 5,565,332).

In some embodiments, humanized antibodies are prepared as described in Queen et al, U.S. Patent Nos: 5,530,101; 5,585,089; 5,693,761; 5,693,762; and 16,180,370.

In some embodiments, the anti-CLEC10A antibodies are human antibodies. Completely "human" anti-CLEC10A antibodies can be desirable for therapeutic treatment of human patients. As used herein, "human antibodies" include antibodies having the amino acid sequence of a human immunoglobulin and include antibodies isolated from human immunoglobulin libraries or from animals transgenic for one or more human immunoglobulin and that do not express endogenous immunoglobulins. Human antibodies can be made by a variety of methods known in the art including phage display methods described above using antibody libraries derived from human immunoglobulin sequences. See U.S. Patent Nos. 4,444,887 and 4,716,111; and PCT publications WO 98/46645; WO 98/50433; WO 98/24893; WO 98/16654; WO 96/34096; WO 96/33735; and WO 97/10741.

Human antibodies can also be produced using transgenic mice which are incapable of expressing functional endogenous immunoglobulins, but which can express human immunoglobulin genes. See, e.g., PCT publications WO 98/24893; WO 92/01047; WO 96/34096; WO 96/33735; U.S. Patent Nos. 5,413,923; 5,625,126; 5,633,425; 5,569,825; 5,661,016; 5,545,806; 5,814,318; 5,885,793; 5,916,771; and 5,939,598.

Completely human antibodies that recognize a selected epitope can be generated using a technique referred to as "guided selection." In this approach a selected non-human monoclonal antibody, e.g., a mouse antibody, is used to guide the selection of a completely human antibody recognizing the same epitope (Jespers et al, 1988, Biotechnology 12:899-903).

In some embodiments, the anti-CLEC10A antibodies are primatized antibodies. The term "primatized antibody" refers to an antibody comprising monkey variable regions and human constant regions. Methods for producing primatized antibodies are known in the art. See e.g., U.S. Patent Nos. 5,658,570; 5,681,722; and 5,693,780.

In some embodiments, the anti-CLEC10A antibodies are bispecific antibodies. Bispecific antibodies are monoclonal, preferably human or humanized, antibodies that have binding specificities for at least two different antigens. In the bispecific antibodies useful in the present methods, the binding specificities can be directed towards two different specific epitopes on CLEC10A, thereby blocking the binding of VWF even more effectively than with a monospecific antibody.

In some embodiments, the anti-CLEC10A antibodies are derivatized antibodies. For example, but not by way of limitation, the derivatized antibodies that have been modified, e.g., by glycosylation, acetylation, pegylation, phosphorylation, amidation, derivatization by known protecting/blocking groups, proteolytic cleavage, linkage to a cellular ligand or other protein (see *infra* for a discussion of antibody conjugates), etc. Any of numerous chemical modifications may be carried out by known techniques, including, but not limited to, specific chemical cleavage, acetylation, formylation, metabolic synthesis of tunicamycin, etc. Additionally, the derivative may contain one or more non-classical amino acids.

In some embodiments, the anti-CLEC10A antibodies or fragments thereof can be antibodies or antibody fragments whose sequence has been modified to reduce at least one constant region-mediated biological effector function relative to the corresponding wild type sequence. To modify an anti-CLEC10A antibody such that it exhibits reduced binding to the Fc receptor, the immunoglobulin constant region segment of the antibody can be mutated at particular regions necessary for Fc receptor (FcR) interactions (See e.g., Canfield and Morrison, 1991, J. Exp. Med. 173 : 1483- 1491; and Lund et al, 1991, J. Immunol. 147:2657-2662). Reduction in FcR binding ability of the antibody can also reduce other effector functions which rely on FcR interactions, such as opsonization and phagocytosis and antigen-dependent cellular cytotoxicity.

In yet another aspect, the anti-CLEC10A antibodies or fragments thereof can be antibodies or antibody fragments that have been modified to increase or reduce their binding affinities to the fetal Fc receptor, FcRn. To alter the binding affinity to FcRn, the immunoglobulin constant region segment of the antibody can be mutated at particular regions necessary for FcRn interactions (See e.g., WO 2005/123780). Increasing the binding affinity to FcRn should increase the antibody's serum half-life, and reducing the binding affinity to FcRn should conversely reduce the antibody's serum half-life. In particular embodiments, the anti-CLEC10A antibody is of the IgG class in which at least one of amino acid residues 250, 314, and 428 of the heavy chain constant region is substituted with an amino acid residue different from that present in the unmodified antibody. The antibodies of IgG class include antibodies of IgG1, IgG2, IgG3, and IgG4. The substitution can be made at position 250, 314, or 428 alone, or in any combinations thereof, such as at positions 250 and 428, or at positions 250 and 314, or at positions 314 and 428, or at positions 250, 314, and 428, with positions 250 and 428 as a preferred combination. For each position, the substituting amino acid can be any amino acid residue different from that present in that position of the unmodified antibody. For position 250, the substituting amino acid residue can be any amino acid residue other than threonine, including, but not limited to, alanine, cysteine, aspartic acid, glutamic acid, phenylalanine, glycine, histidine, isoleucine, lysine, leucine, methionine, asparagine, proline, glutamine, arginine, serine, valine, tryptophan, or tyrosine. For position 314, the substituting amino acid residue can be any amino acid residue other than leucine, including, but not limited to, alanine, cysteine, aspartic acid, glutamic acid, phenylalanine, glycine, histidine, isoleucine, lysine, methionine, asparagine, proline, glutamine, arginine, serine, threonine, valine, tryptophan, or tyrosine. For position 428, the substituting amino acid residues can be any amino acid residue other than methionine, including, but not limited to, alanine, cysteine, aspartic acid, glutamic acid, phenylalanine, glycine, histidine, isoleucine, lysine, leucine, asparagine, proline, glutamine, arginine, serine, threonine, valine, tryptophan, or tyrosine. Specific combinations of suitable amino acid substitutions are identified in Table 1 of WO 2005/123780.

See also, Hinton et ah, US Patent Nos. 7,217,797, 7,361,740, 7,365,168, and 7,217,798.

In yet other aspects, an anti-CLEC10A antibody has one or more amino acids inserted into one or more of its hypervariable region, for example as described in US 2007/0280931.

### Antibody Conjugates

In some embodiments, the anti-CLEC10A antibodies are antibody conjugates that are modified, e.g., by the covalent attachment of any type of molecule to the antibody, such that covalent attachment does not interfere with binding to CLEC10A. Techniques for conjugating effector moieties to antibodies are well known in the art (See, e.g., Hellstrom et ah, Controlled Drag Delivery, 2nd Ed., at pp. 623-53 (Robinson et ah, eds., 1987)); Thorpe et ah, 1982, Immunol. Rev. 62: 119-58 and Dubowchik eï α/., 1999, Pharmacology and Therapeutics 83:67-123).

In one example, the antibody or fragment thereof is fused via a covalent bond (e.g., a peptide bond), at optionally the N-terminus or the C-terminus, to an amino acid sequence of another protein (or portion thereof; preferably at least a 10, 20 or 50 amino acid portion of the protein). Preferably the antibody, or fragment thereof, is linked to the other protein at the N-terminus of the constant domain of the antibody. Recombinant DNA procedures can be used to create such fusions, for example as described in WO 86/01533 and EP0392745. In another example the effector molecule can increase half-life in vivo. Examples of suitable effector molecules of this type include polymers, albumin, albumin binding proteins or albumin binding compounds such as those described in WO 2005/117984.

In some embodiments, anti-CLEC10A antibodies can be attached to poly(ethyleneglycol) (PEG) moieties. For example, if the antibody is an antibody fragment, the PEG moieties can be attached through any available amino acid side-chain or terminal amino acid functional group located in the antibody fragment, for example any free amino, imino, thiol, hydroxyl or carboxyl group. Such amino acids can occur naturally in the antibody fragment or can be engineered into the fragment using recombinant DNA methods. See for example U.S. Patent No. 5,219,996. Multiple sites can be used to attach two or more PEG molecules. Preferably PEG moieties are covalently linked through a thiol group of at least one cysteine residue located in the antibody fragment. Where a thiol group is used as the point of attachment, appropriately activated effector moieties, for example thiol selective derivatives such as maleimides and cysteine derivatives, can be used.

In another example, an anti-CLEC10A antibody conjugate is a modified Fab' fragment which is PEGylated, i.e., has PEG (poly(ethyleneglycol)) covalently attached thereto, e.g., according to the method disclosed in EP0948544. See also Poly(ethyleneglycol) Chemistry, Biotechnical and Biomedical Applications, (J. Milton Harris (ed.), Plenum Press, New York, 1992); Poly(ethyleneglycol) Chemistry and Biological Applications, (J. Milton Harris and S. Zalipsky, eds., American Chemical Society, Washington D. C, 1997); and Bioconjugation Protein Coupling Techniques for the Biomedical Sciences, (M. Aslam and A. Dent, eds., Grove Publishers, New York, 1998); and Chapman, 2002, Advanced Drug Delivery Reviews 54:531- 545.

### Treatment of coagulation disorder

The anti-CLEC10A antibodies described herein are useful for treating coagulation disorders including, but not limited to, hemophilia and von Willebrand disease. Preferably, the disease is hemophilia A or von Willebrand disease.

The term "hemophilia A" refers to a deficiency in functional coagulation FVIII, which is usually inherited.

The term "von Willebrand disease" (VWD) refers to a coagulation abnormality associated with a qualitative or quantitative deficiency of VWF.

Treatment of a disease encompasses the treatment of patients already diagnosed as having any form of the disease at any clinical stage or manifestation; the delay of the onset or evolution or aggravation or deterioration of the symptoms or signs of the disease; and/or preventing and/or reducing the severity of the disease.

A "subject" or "patient" to whom an anti-CLEC10A antibody is administered can be a mammal, such as a non-primate (e.g., cow, pig, horse, cat, dog, rat, etc.) or a primate (e.g., monkey or human). Preferably the patient is a human. In certain aspects, the human is a pediatric patient. In other aspects, the human is an adult patient.

Compositions comprising an anti-CLEC10A antibody and, optionally one or more additional therapeutic agents, such as the second therapeutic agents described below, are described herein. The compositions typically are supplied as part of a sterile, pharmaceutical composition that includes a pharmaceutically acceptable carrier. This composition can be in any suitable form (depending upon the desired method of administering it to a patient).

The anti-CLEC10A antibodies can be administered to a patient by a variety of routes such as orally, transdermally, subcutaneously, intranasally, intravenously, intramuscularly, intrathecally, topically or locally. The most suitable route for administration in any given case will depend on the particular antibody, the subject, and the nature and severity of the disease and the physical condition of the subject. Typically, an anti-CLEC10A antibody will be administered intravenously.

In typical embodiments, an anti-CLEC10A antibody is present in a pharmaceutical composition at a concentration sufficient to permit intravenous administration at 0.5 mg/kg to 20 mg/kg. In some embodiments, the concentration of antibody suitable for use in the compositions and methods described herein includes, but is not limited to, 0.5 mg/kg, 0.75 mg/kg, 1 mg/kg, 2 mg/kg, 2.5 mg/kg, 3 mg/kg, 4 mg/kg, 5 mg/kg, 6 mg/kg, 7 mg/kg, 8 mg/kg, 9 mg/kg, 10 mg/kg, 11 mg/kg, 12 mg/kg, 13 mg/kg, 14 mg/kg, 15 mg/kg, 16 mg/kg, 17 mg/kg, 18 mg/kg, 19 mg/kg, 20 mg/kg, or a concentration ranging between any of the foregoing values, e.g., 1 mg/kg to 10 mg/kg, 5 mg/kg to 15 mg/kg, or 10 mg/kg to 18 mg/kg.

The effective dose of an anti-CLEC10A antibody can range from about 0.001 to about 750 mg/kg per single (e.g., bolus) administration, multiple administrations or continuous administration, or to achieve a serum concentration of 0.01-5000 µg/ml serum concentration per single (e.g., bolus) administration, multiple administrations or continuous administration, or any effective range or value therein depending on the condition being treated, the route of administration and the age, weight and condition of the subject. In certain embodiments, each dose can range from about 0.5 mg to about 50 mg per kilogram of body weight or from about 3 mg to about 30 mg per kilogram body weight. The antibody is can be formulated as an aqueous solution.

Pharmaceutical compositions can be conveniently presented in unit dose forms containing a predetermined amount of an anti-CLEC10A antibody per dose. Such a unit can contain 0.5 mg to 5 g, for example, but without limitation, 1 mg, 10 mg, 20 mg, 30 mg, 40 mg, 50 mg, 100 mg, 200 mg, 300 mg, 400 mg, 500 mg, 750 mg, 1000 mg, or any range between any two of the foregoing values, for example 10 mg to 1000 mg, 20 mg to 50 mg, or 30 mg to 300 mg. Pharmaceutically acceptable carriers can take a wide variety of forms depending, e.g., on the condition to be treated or route of administration.

Determination of the effective dosage, total number of doses, and length of treatment with an anti-CLEC10A antibody is well within the capabilities of those skilled in the art, and can be determined using a standard dose escalation study.

Therapeutic formulations of the anti-CLEC10A antibodies suitable in the methods described herein can be prepared for storage as lyophilized formulations or aqueous solutions by mixing the antibody having the desired degree of purity with optional pharmaceutically-acceptable carriers, excipients or stabilizers typically employed in the art (all of which are referred to herein as "carriers"), i.e., buffering agents, stabilizing agents, preservatives, isotonifiers, non- ionic detergents, antioxidants, and other miscellaneous additives. See, Remington's Pharmaceutical Sciences, 16th edition (Osol, ed. 1980). Such additives must be nontoxic to the recipients at the dosages and concentrations employed.

Buffering agents help to maintain the pH in the range which approximates physiological conditions. They can be present at concentrations ranging from about 2 mM to about 50 mM. Suitable buffering agents include both organic and inorganic acids and salts thereof such as citrate buffers (e.g., monosodium citrate-disodium citrate mixture, citric acid-trisodium citrate mixture, citric acid-monosodium citrate mixture, etc.), succinate buffers (e.g., succinic acid-monosodium succinate mixture, succinic acid-sodium hydroxide mixture, succinic acid-disodium succinate mixture, etc.), tartrate buffers (e.g., tartaric acid-sodium tartrate mixture, tartaric acid-potassium tartrate mixture, tartaric acid-sodium hydroxide mixture, etc.), fumarate buffers (e.g., fumaric acid-monosodium fumarate mixture, fumaric acid-disodium fumarate mixture, monosodium fumarate-disodium fumarate mixture, etc.), gluconate buffers (e.g., gluconic acid-sodium glyconate mixture, gluconic acid-sodium hydroxide mixture, gluconic acid-potassium glyuconate mixture, etc.), oxalate buffer (e.g., oxalic acid-sodium oxalate mixture, oxalic acid-sodium hydroxide mixture, oxalic acid-potassium oxalate mixture, etc), lactate buffers (e.g., lactic acid-sodium lactate mixture, lactic acid-sodium hydroxide mixture, lactic acid-potassium lactate mixture, etc.) and acetate buffers (e.g., acetic acid-sodium acetate mixture, acetic acid-sodium hydroxide mixture, etc.). Additionally, phosphate buffers, histidine buffers and trimethylamine salts such as Tris can be used.

Preservatives can be added to retard microbial growth, and can be added in amounts ranging from 0.2%- 1% (w/v). Suitable preservatives include phenol, benzyl alcohol, meta-cresol, methyl paraben, propyl paraben, octadecyldimethylbenzyl ammonium chloride, benzalconium halides (e.g., chloride, bromide, and iodide), hexamethonium chloride, and alkyl parabens such as methyl or propyl paraben, catechol, resorcinol, cyclohexanol, and 3-pentanol. Isotonicifiers sometimes known as "stabilizers" can be added to ensure isotonicity of liquid compositions and include polhydric sugar alcohols, preferably trihydric or higher sugar alcohols, such as glycerin, erythritol, arabitol, xylitol, sorbitol and mannitol. Stabilizers refer to a broad category of excipients which can range in function from a bulking agent to an additive which solubilizes the therapeutic agent or helps to prevent denaturation or adherence to the container wall. Typical stabilizers can be polyhydric sugar alcohols (enumerated above); amino acids such as arginine, lysine, glycine, glutamine, asparagine, histidine, alanine, ornithine, L-leucine, 2-phenylalanine, glutamic acid, threonine, etc., organic sugars or sugar alcohols, such as lactose, trehalose, stachyose, mannitol, sorbitol, xylitol, ribitol, myoinisitol, galactitol, glycerol and the like, including cyclitols such as inositol; polyethylene glycol; amino acid polymers; sulfur containing reducing agents, such as urea, glutathione, thioctic acid, sodium thioglycolate, thioglycerol, α-monothioglycerol and sodium thio sulfate; low molecular weight polypeptides (e.g., peptides of 10 residues or fewer); proteins such as human serum albumin, bovine serum albumin, gelatin or immunoglobulins; hydrophylic polymers, such as polyvinylpyrrolidone monosaccharides, such as xylose, mannose, fructose, glucose; disaccharides such as lactose, maltose, sucrose and trisaccacharides such as raffinose; and polysaccharides such as dextran. Stabilizers can be present in the range from 0.1 to 10,000 weights per part of weight active protein.

Non-ionic surfactants or detergents (also known as "wetting agents") can be added to help solubilize the therapeutic agent as well as to protect the therapeutic protein against agitation-induced aggregation, which also permits the formulation to be exposed to shear surface stressed without causing denaturation of the protein. Suitable non-ionic surfactants include polysorbates (20, 80, etc.), polyoxamers (184, 188 etc.), Pluronic polyols, polyoxyethylene sorbitan monoethers (TWEEN®-20, TWEEN®-80, etc.). Non-ionic surfactants can be present in a range of about 0.05 mg/ml to about 1.0 mg/ml, or in a range of about 0.07 mg/ml to about 0.2 mg/ml.

Additional miscellaneous excipients include bulking agents (e.g., starch), chelating agents (e.g., EDTA), antioxidants (e.g., ascorbic acid, methionine, vitamin E), and cosolvents.

The formulation herein can also contain a second therapeutic agent in addition to an anti-CLEC10A antibody. Examples of suitable second therapeutic agents are provided below.

The dosing schedule can vary from once a month to daily depending on a number of clinical factors, including the type of disease, severity of disease, and the patient's sensitivity to the anti-CLEC10A antibody. In specific embodiments, an anti-CLEC10A antibody is administered daily, twice weekly, three times a week, every 5 days, every 10 days, every two weeks, every three weeks, every four weeks or once a month, or in any range between any two of the foregoing values, for example from every four weeks to every month, from every 10 days to every two weeks, or from two to three times a week, etc.

The dosage of an anti-CLEC10A antibody to be administered will vary according to the particular antibody, the subject, and the nature and severity of the disease, the physical condition of the subject, the therapeutic regimen (e.g., whether a second therapeutic agent is used), and the selected route of administration; the appropriate dosage can be readily determined by a person skilled in the art.

It will be recognized by one of skill in the art that the optimal quantity and spacing of individual dosages of an anti-CLEC10A antibody will be determined by the nature and extent of the condition being treated, the form, route and site of administration, and the age and condition of the particular subject being treated, and that a physician will ultimately determine appropriate dosages to be used. This dosage can be repeated as often as appropriate. If side effects develop the amount and/or frequency of the dosage can be altered or reduced, in accordance with normal clinical practice.

### Combination Therapy

Preferably, the patient being treated with the anti-CLEC10A antibody is also treated with a conventional therapy of coagulation disorders. For example, a patient suffering from hemophilia is typically also being treated with a blood coagulation factor, e.g. Factor VIII, VWF or combinations thereof.

The term "von Willebrand factor" (VWF) as used herein includes naturally occurring VWF, but also variants thereof, e.g. fragments, fusion proteins or conjugates, or sequence variants where one or more residues have been inserted, deleted or substituted, retaining the biological activity of naturally occurring VWF. The biological activity is retained in the sense of the invention if the VWF variant retains at least 10%, preferably at least 25%, more preferably at least 50%, most preferably at least 75% of at least one of the biological activities of wild-type VWF. The biological activity of wild-type VWF and variants thereof can be determined by the artisan using methods for ristocetin co-factor activity (Federici A B et al. 2004. Haematologica 89:77-85), binding of VWF to GP Ibα of the platelet glycoprotein complex Ib-V-IX (Sucker et al. 2006. Clin Appl Thromb Hemost. 12:305-310), a collagen binding assay (Kallas & Talpsep. 2001. Annals of Hematology 80:466-471), or binding to Factor VIII.

The terms "Factor VIII" and "FVIII" are used synonymously herein. "FVIII" includes natural allelic variations of FVIII that may exist and occur from one individual to another. FVIII may be plasma-derived or recombinantly produced, using well known methods of production and purification. The degree and location of glycosylation, tyrosine sulfation and other post-translation modifications may vary, depending on the chosen host cell and its growth conditions.

The term FVIII includes FVIII analogues. The term "FVIII analogue" as used herein refers to a FVIII molecule (full-length or B-domain-truncated/deleted, or single chain FVIII) wherein one or more amino acids have been substituted or deleted compared to the wild type amino acid sequence of FVIII (i.e. the sequence defined by UniProt identifier P00451) or, for B-domain truncated/deleted FVIII molecules, the corresponding part of that amino acid sequence. FVIII analogues do not occur in nature but are obtained by human manipulation.

The Factor VIII molecules used according to the present invention may also be B-domain-truncated/deleted FVIII molecules wherein the remaining domains correspond to the sequences as set forth in amino acid numbers 1-740 and 1649-2332 of the FVIII wild type amino acid sequence. Other forms of B-domain deleted FVIII molecules have additionally a partial deletion in their a3 domain, which leads to single-chain FVIII molecules.

It follows that these FVIII molecules are recombinant molecules produced in transformed host cells, preferably of mammalian origin. However, the remaining domains in a B-domain deleted FVIII, (i.e. the three A- domains, the two C-domains and the a1, a2 and a3 regions) may differ slightly e.g. about 1%, 2%, 3%, 4% or 5% from the respective wild type amino acid sequence (amino acids 1-740 and 1649-2332).

The FVIII molecules used in accordance with the present invention may be two-chain FVIII molecules or single-chain FVIII molecules. The FVIII molecules included in the composition of the present invention may also be biologically active fragments of FVIII, i.e., FVIII wherein domain(s) other than the B-domain has/have been deleted or truncated, but wherein the FVIII molecule in the deleted/truncated form retains its ability to support the formation of a blood clot. FVIII activity can be assessed in vitro using techniques well known in the art. A preferred test for determining FVIII activity according to this invention is the chromogenic substrate assay or the one stage assay (see infra). Amino acid modifications (substitutions, deletions, etc.) may be introduced in the remaining domains, e.g., in order to modify the binding capacity of Factor VIII with various other components such as e.g. Von Willebrand Factor (vWF), low density lipoprotein receptor-related protein (LPR), various receptors, other coagulation factors, cell surfaces, etc. or in order to introduce and/or abolish glycosylation sites, etc. Other mutations that do not abolish FVIII activity may also be accommodated in a FVIII molecule/analogue for use in a composition of the present invention.

FVIII analogues also include FVIII molecules, in which one or more of the amino acid residues of the parent polypeptide have been deleted or substituted with other amino acid residues, and/or wherein additional amino acid residues has been added to the parent FVIII polypeptide.

Furthermore, the Factor VIII molecules/analogues may comprise other modifications in e.g. the truncated B-domain and/or in one or more of the other domains of the molecules ("FVIII derivatives"). These other modifications may be in the form of various molecules conjugated to the Factor VIII molecule, such as e.g. polymeric compounds, peptidic compounds, fatty acid derived compounds, etc.

The term FVIII includes glycopegylated FVIII. In the present context, the term "glycopegylated FVIII" is intended to designate a Factor VIII molecule (including full length FVIII and B-domain truncated/deleted FVIII) wherein one or more PEG group(s) has/have been attached to the FVIII polypeptide via the polysaccharide sidechain(s) (glycan(s)) of the polypeptide.

The term FVIII includes FVIII molecules having protective groups or half-life extending moieties. The terms "protective groups"/"half-life extending moieties" is herein understood to refer to one or more chemical groups attached to one or more amino acid site chain functionalities such as -SH, -OH, -COOH, -CONH2, -NH2, or one or more N- and/or O-glycan structures and that can increase in vivo circulatory half-life of a number of therapeutic proteins/peptides when conjugated to these proteins/peptides. Examples of protective groups/half-life extending moieties include: Biocompatible fatty acids and derivatives thereof, Hydroxy Alkyl Starch (HAS) e.g. Hydroxy Ethyl Starch (HES), Poly (Glyx-Sery)n (Homo Amino acid Polymer (HAP)), Hyaluronic acid (HA), Heparosan polymers (HEP), Phosphorylcholine-based polymers (PC polymer), Fleximer® polymers (Mersana Therapeutics, MA, USA), Dextran, Poly-sialic acids (PSA), polyethylene glycol (PEG), an Fc domain, Transferrin, Albumin, Elastin like peptides, XTEN® polymers (Amunix, CA, USA), Albumin binding peptides, a von Willebrand factor fragment (vWF fragment), a Carboxyl Terminal Peptide (CTP peptide, Prolor Biotech, IL), and any combination thereof (see, for example, McCormick, C.L., A.B. Lowe, and N. Ayres, Water-Soluble Polymers, in Encyclopedia of Polymer Science and Technology. 2002, John Wiley & Sons, Inc.). The manner of derivatization is not critical and can be elucidated from the above.

The FVIII molecules which can be used in accordance with this invention include fusion proteins comprising a FVIII amino acid sequence fused to a heterologous amino acid sequence, preferably a half-life extending amino acid sequence. Preferred fusion proteins are Fc fusion proteins and albumin fusion proteins. The term "Fc fusion protein" is herein meant to encompass FVIII fused to an Fc domain that can be derived from any antibody isotype. An IgG Fc domain will often be preferred due to the relatively long circulatory half-life of IgG antibodies. The Fc domain may furthermore be modified in order to modulate certain effector functions such as e.g. complement binding and/or binding to certain Fc receptors. Fusion of FVIII with an Fc domain, which has the capacity to bind to FcRn receptors, will generally result in a prolonged circulatory half-life of the fusion protein compared to the half-life of the wt FVIII. It follows that a FVIII molecule for use in the present invention may also be a derivative of a FVIII analogue, such as, for example, a fusion protein of an FVIII analogue, a PEGylated or glycoPEGylated FVIII analogue, or a FVIII analogue conjugated to a heparosan polymer. The term "albumin fusion protein" is herein meant to encompass FVIII fused to an albumin amino acid sequence or a fragment or derivative thereof. The heterologous amino acid sequence may be fused to the N- or C-terminus of FVIII, or it may be inserted internally within the FVIII amino acid sequence. The heterologous amino acid sequence may be any "half life extending polypeptide" described in WO 2008/077616 A1.

Examples of FVIII molecules for use in compositions of the present invention comprise for instance the FVIII molecules described in WO 2010/045568, WO 2009/062100, WO 2010/014708, WO 2008/082669, WO 2007/126808, US 2010/0173831, US 2010/0173830, US 2010/0168391, US 2010/0113365, US 2010/0113364, WO 2003/031464, WO 2009/108806, WO 2010/102886, WO 2010/115866, WO 2011/101242, WO 2011/101284, WO 2011/101277, WO 2011/131510, WO 2012/007324, WO 2011/101267, WO 2013/083858, and WO 2004/067566.

Examples of FVIII molecules, which can be used in a composition of the present invention include the active ingredient of Advate®, Helixate®, Kogenate®, Xyntha® as well as the FVIII molecule described in WO 2008/135501, WO 2009/007451 and the construct designated "dBN(64-53)" of WO 2004/067566.

The concentration of Factor VIII in the composition used according to the present invention is typically in the range of 10-10,000 IU/mL. In different embodiments, the concentration of FVIII molecules in the compositions of the invention is in the range of 10-8,000 IU/mL, or 10-5,000 IU/mL, or 20-3,000 IU/mL, or 50-1,500 IU/mL, or 3,000 IU/mL, or 2,500 IU/mL, or 2,000 IU/mL, or 1,500 IU/mL, or 1,200 IU/mL, 1,000 IU/mL, or 800 IU/mL, or 600 IU/mL, or 500 IU/mL, or 400 IU/mL, or 300 IU/mL, or 250 IU/mL, or 200 IU/mL, or 150 IU/mL, or 100 IU/mL.

"International Unit," or "IU," is a unit of measurement of the blood coagulation activity (potency) of FVIII as measured by a FVIII activity assay such as a one stage clotting assay or a chromogenic substrate FVIII activity assay using a standard calibrated against an international standard preparation calibrated in "IU". One stage clotting assays are known to the art, such as that described in N Lee, Martin L, et al., An Effect of Predilution on Potency Assays of FVIII Concentrates, Thrombosis Research (Pergamon Press Ltd.) 30, 511 519 (1983). Principle of the one stage assay: The test is executed as a modified version of the activated Partial Thromboplastin Time (aPTT)-assay: Incubation of plasma with phospholipids and a surface activator leads to the activation of factors of the intrinsic coagulation system. Addition of calcium ions triggers the coagulation cascade. The time to formation of a measurable fibrin clot is determined. The assay is executed in the presence of Factor VIII deficient plasma. The coagulation capability of the deficient plasma is restored by Coagulation Factor VIII included in the sample to be tested. The shortening of coagulation time is proportional to the amount of Factor VIII present in the sample. The activity of Coagulation Factor VIII is quantified by direct comparison to a standard preparation with a known activity of Factor VIII in International Units.

Another standard assay is a chromogenic substrate assay. Chromogenic substrate assays may be purchased commercially, such as the coamatic FVIII test kit (Chromogenix-Instrumentation Laboratory SpA V. le Monza 338 - 20128 Milano, Italy). Principle of the chromogenic assay: In the presence of calcium and phospholipid, Factor X is activated by Factor IXa to Factor Xa. This reaction is stimulated by Factor Villa as cofactor. FVIIIa is formed by low amounts of thrombin in the reaction mixture from FVIII in the sample to be measured. When using the optimum concentrations of Ca2+, phospholipid and Factor IXa and an excess quantity of Factor X, activation of Factor X is proportional to the potency of Factor VIII. Activated Factor X releases the chromophore pNA from the chromogenic substrate S-2765. The release of pNA, measured at 405 nm, is therefore proportional to the amount of FXa formed, and, therefore, also to the Factor VIII activity of the sample.

In one embodiment, the treatment comprises administering the anti-CLEC10A antibody of the invention and Factor VIII to a patient suffering from hemophilia, preferably hemophilia A.

In another embodiment, the treatment comprises administering the anti-CLEC10A antibody of the invention and VWF to a patient suffering from hemophilia, preferably hemophilia A.

In another embodiment, the treatment comprises administering the anti-CLEC10A antibody of the invention and Factor VIII and VWF to a patient suffering from hemophilia, preferably hemophilia A.

In another embodiment, the treatment comprises administering the anti-CLEC10A antibody of the invention and VWF to a patient suffering from von Willebrand disease.

In a particular embodiment, the anti-CLEC10A antibody and the blood coagulation factor (e.g. Factor VIII, VWF or combinations thereof) are administered simultaneously. In another embodiment, the anti-CLEC10A antibody and the blood coagulation factor (e.g. Factor VIII, VWF or combinations thereof) are administered separately. The time between the administration of the anti-CLEC10A antibody and the blood coagulation factor (e.g. Factor VIII, VWF or combinations thereof) is not particularly limited. It is preferred that the blood coagulation factor (e.g. Factor VIII, VWF or combinations thereof) is administered prior to the anti-CLEC10A antibody.

Another aspect of the present invention is a pharmaceutical kit comprising (i) a first compound (preferably an antibody) as defined hereinabove and (ii) a polypeptide selected from the group consisting of Factor VIII, von Willebrand factor and combinations thereof. Preferably, the compound (preferably the antibody) and the polypeptide are contained in separate compositions.

Another aspect of the present invention is a pharmaceutical kit comprising (i) a first compound (preferably an antibody) as defined hereinabove and (ii) a polypeptide selected from the group consisting of Factor VIII, von Willebrand factor and combinations thereof, for simultaneous, separate or sequential use in the treatment of a blood coagulation disorder.

Another aspect of the invention is the use of a compound (preferably an antibody) as defined hereinabove for increasing the half-life or reducing the clearance of von Willebrand Factor.

The term "half-life" refers to the time it takes to eliminate half of the protein from the circulation *in vivo.* The area under the curve (AUC) can be determined to assess clearance effects. A reduction in clearance leads to higher AUC values and to an increase in half-life.

Yet another aspect of the invention is the use of a compound (preferably an antibody) as defined hereinabove for increasing the half-life of Factor VIII.

Yet another aspect of the invention is a compound (preferably an antibody) as defined hereinabove for use in prolonging the half-life of von Willebrand factor in a therapeutic treatment.

The invention further relates to a method of increasing the half-life or reducing the clearance of von Willebrand Factor *in vivo,* comprising administering to a subject an effective amount of a compound (preferably an antibody) as defined hereinabove.

A further aspect of this disclosure is a method of treating a blood coagulation disorder, comprising administering to a patient in need thereof an effective amount of a compound (preferably an antibody) as defined hereinabove.

A further aspect is the use of a compound (preferably an antibody) as defined hereinabove for reducing the frequency of administration of FVIII in a treatment of hemophilia A. The frequency of intravenous or subcutaneous administration of FVIII may be reduced to twice per week. Alternatively, the frequency of intravenous or subcutaneous administration of FVIII may be reduced to once per week.

A further aspect is the use of a compound (preferably an antibody) as defined hereinabove for reducing the frequency of administration of VWF in a treatment of VWD. The frequency of intravenous or subcutaneous administration of VWF may be reduced to twice per week. Alternatively, the frequency of intravenous or subcutaneous administration of VWF may be reduced to once per week.

Another aspect is the use of a compound (preferably an antibody) as defined hereinabove for reducing the dose FVIII to be administered in a treatment of hemophilia A.

Another aspect is the use of a compound (preferably an antibody) as defined hereinabove for reducing the dose VWF to be administered in a treatment of VWD.

The term "ABO(H) blood group antigen", as used herein, refers to carbohydrate antigens present on erythrocytes that are commonly recognized by anti-A or anti-B antibodies. The ABO(H) blood group system is the most important blood type system in human blood transfusion. The H-antigen is an essential precursor to the ABO(H) blood group antigens, and is a carbohydrate structure linked mainly to protein, with a minor fraction attached to ceramide. It consists of a chain of β-D-galactose, β-D-N-acetylglucosamine, β-D-galactose, and 2-linked a-L-fucose. The A-antigen contains an a-N-acetylgalactosamine bonded to the D-galactose residue at the end of the H-antigen, whereas the B-antigen has an a-D-galactose residue bonded to the D-galactose of the H-antigen. Therefore, the terminal sugar residues of the ABO(H) blood group system are galactose, N-acetylgalactosamine and fucose.

### EXAMPLES

### Example 1: Interaction of Monomeric Human VWF with Recombinant Human CLEC10A

### Materials & Methods

Surface plasmon resonance (SPR) technology (Biacore T200, GE Healthcare Biosciences, Uppsala, Sweden) was applied to evaluate mechanisms of real-time biomolecular interactions between purified monomeric human VWF (analyte) and receptor proteins such as CLEC10A (ligand). SPR based instruments, such as the Biacore T200, use an optical method to monitor the change in refractive index close to the backside of a metal sensor surface to which a ligand is immobilized. The analyte is in the mobile phase that is continuously passed over the immobilized ligand. The event of capturing the analyte by the ligand leads to an accumulation of analyte on the surface and results in an increase in the refractive index which is measured as an SPR response in real time by detecting changes in the intensity of the reflected light. The SPR signal is expressed in RU and the change in signal over time is displayed as a sensorgram. Background responses from a reference flow cell are subtracted from the experimental responses. The size of the change in SPR signal is directly proportional to the mass being immobilized or captured, and allows assay of binding constants and kinetic analysis of binding phenomena in real-time and in a label-free environment (Biacore Handbook, 2008; Schasfoort & Tudos, 2008; Biacore Handbook, 2012).

Interaction experiments were performed at a flow cell temperature of +25°C by applying running buffer containing 10 mM HEPES, 150 mM NaCl, 5 mM CaCl2 and 0.05% (w/v) Tween-20 at pH 7.4, which was also used as sample dilution buffer. The proteins used were transferred into running buffer by PD-10 desalting columns (GE Healthcare Life Sciences, Freiburg, Germany) prior to application. Reagents and buffer stock solutions were purchased from GE Healthcare Biosciences (Uppsala, Sweden) and buffer solutions were sterile filtered (0.22 µm Stericup filter units, Millipore, Massachusetts, USA) prior to use. The extracellular domain of CLEC10A was acquired from R&D Systems (Wiesbaden, Germany). Furthermore, human albumin (CSL Behring, Marburg, Germany) was used as control protein. CLEC10A was captured on a Series S Sensor Chip C1 (flat carboxymethylated) pretreated according to the manufacturer's instructions. The investigation of CLEC10A was started with a preconcentration step in order to estimate the amount of protein required to obtain a desired level of immobilization as well as to determine the optimal pH value for immobilization. For an efficient immobilization the pH value of the immobilization buffer should be lower than the isoelectric point of the ligand. Thus, for pH scouting, CLEC10A was first dissolved in WFI to a concentration of 1 mg/mL and further 1:50 diluted in 10 mM sodium acetate buffer of pH 4.0, 4.5, 5.0 and 5.5, respectively. The method was performed according to the immobilization pH scouting wizard in the Biacore instrument control software by applying a contact time of 180 seconds and a flow rate of 5 µL/min. After analysis, the surface was regenerated with 50 mM NaOH before covalent immobilization was started.

For immobilization purpose, dissolved CLEC10A was diluted with 10 mM sodium acetate buffer of the optimum pH value (pH 5.0) to a concentration of 20 µg/mL. The ligand was covalently immobilized through free amine groups to the carboxymethylated dextran matrix by applying the amine coupling kit according to the manufacturer's protocol. Coupling occurs between primary amine groups of the ligand and free carboxylic acid groups present on the chip surface after its activation with a 1:1 mixture of 0.05 M EDC and 0.2 M NHS for 7 min. Immobilization was performed at a flow rate of 10 µL/min at +25°C. Ultimately, a surface density range between 700 and 1,500 RU was targeted. In addition, a blank flow cell without immobilized protein was included as a reference surface on the chip for bulk shift and nonspecific binding changes. After ligand immobilization, both chip surfaces were blocked by 1 M ethanolamine-HCI (pH 8.5) for 7 min and non-covalent nonspecific interactions potentially formed during the immobilization process were removed by washing with 10 mM NaOH for 10 seconds 3 times at a flow rate of 25 µL/min. The SPR baseline was conditioned by performing 5 startup cycles with running buffer in each case.

Increasing concentrations of monomeric VWF were prepared as a 2-fold serial dilution series in running buffer and were sequentially injected across the chip surface at 25 µL/min in order to characterize protein-ligand interaction. The concentrations of VWF monomers ranged between 4,000 and 31.25 nM and were calculated based on the MW of monomeric VWF. All samples were designed to contain similar buffer compositions due to the high sensitivity of the SPR system to changes in buffer composition. The relatively high flow rate was chosen to avoid potential rebinding due to mass transfer limitations. Interaction analysis cycles consisted of a 5 min sample injection phase. In this association phase, VWF bound to CLEC10A immobilized on the surface, and increased the surface mass. This phase was followed by a dissociation phase of 17 min in running buffer. In the dissociation phase, the sample was replaced by running buffer and the dissociated VWF was removed from the surface, resulting in a decreased surface mass. All samples were tested as repeat determination. Both the chip surface and the control surface were regenerated with a 10 second pulse of 10 mM NaOH between each run in order to remove bound VWF from surface-immobilized CLEC10A, a step that was repeated 3 times before starting a final 2 min wash step with running buffer and the next run. Although kinetic data were analyzed using Biacore T200 Evaluation Software Version 1.0 (GE Healthcare Biosciences, Uppsala, Sweden), the data set was only used for information purpose.

SPR was predominantly used as mass detector. An interaction of VWF with CLEC10A was detected by an increase in accumulating mass and specific binding was identified by subtracting the binding response recorded from the control surface, followed by subtracting an average of the buffer blank injections. A time point was positioned 20 seconds after the end of sample injection and was evaluated as representative for a stable protein-ligand interaction, which was of interest. Thus, this point was used for the assessment and calculation of biomolecular interactions between VWF and CLEC10A. Furthermore, testing was performed at least in duplicate and the response was calculated relative to the baseline in each case. In addition to the general assessment of the VWF-CLEC10A interaction, affinity constants (R50%) were determined, representing the response of the total VWF concentration which would occupy 50% of CLEC10A. Affinity constants were used for binding affinity estimation by applying the defined report point and were derived from nonlinear global curve fitting using the steady state affinity fit preset by the software. Moreover, dissociation rate constants (off-rate) were calculated by fitting the dissociation phase alone. A suitable dissociation model was established (Biacore Training Courses, 2008) and the report point defined earlier was used for the calculation.

### Results

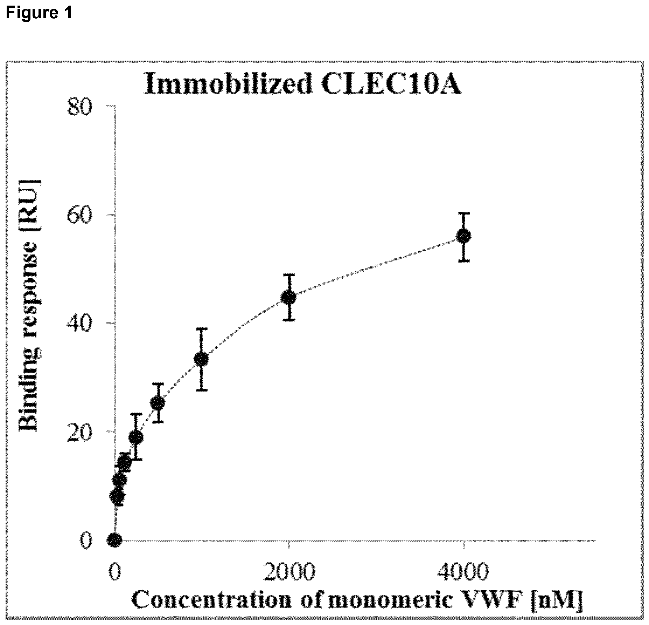

For CLEC10A characterized by SPR, VWF exhibited a strong binding in a dose-dependent manner, as shown in Figure 1.

### Example 2: Interaction of Monomeric Human VWF with Both Recombinant Human CLEC10A and the CLEC10A Orthologous Mouse Proteins (MGL1 and MGL2)

### Materials & Methods

CLEC10A, MGL1 and MGL2 were immobilized on a Series S Sensor Chip CM3 (pH value for immobilization: pH 5.0 for both CLEC10A and MGL1; pH 5.5 for MGL2), respectively, as described in Example 1. Immobilization of the receptor proteins was performed by amine coupling, but a surface density of 6,000 (±500) RU was targeted. Testing was performed as described in Example 1.

### Results

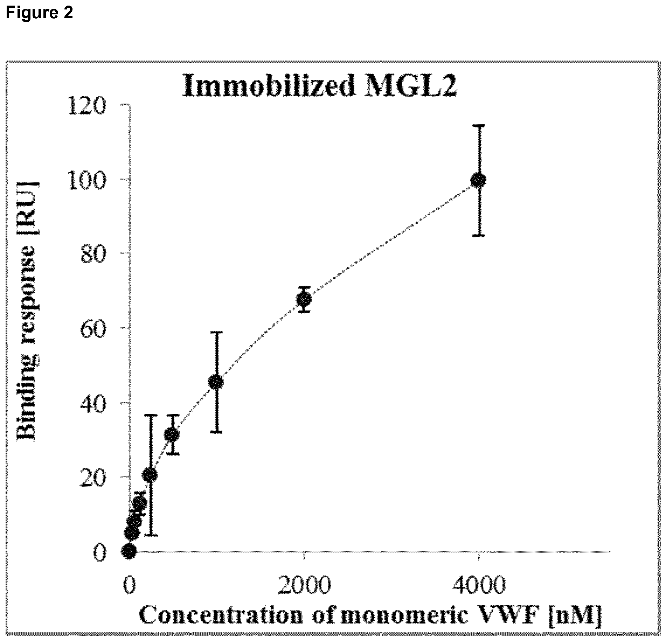

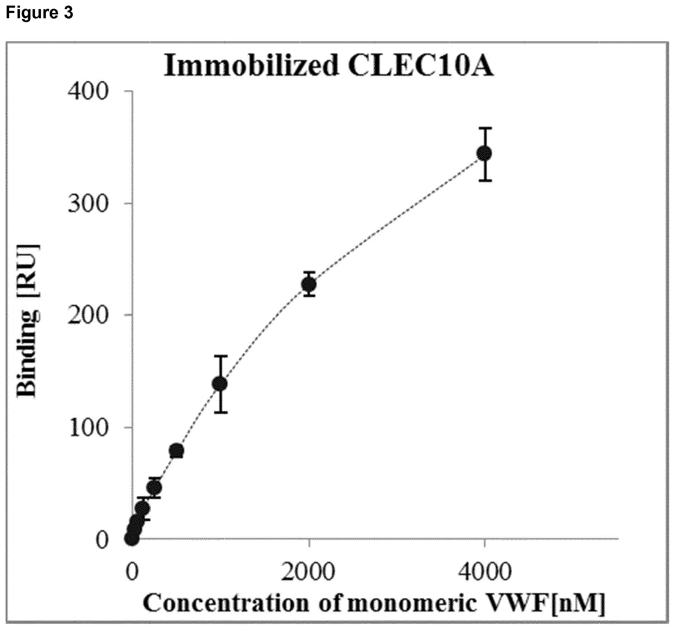

Binding interactions were investigated by SPR analysis. The results in Table 1 and in Figures 2 and 3 clearly demonstrated that purified human VWF monomers bound to human CLEC10A and both murine receptor proteins in a dose-dependent manner in vitro. In general, similar binding characteristics were observed for all three receptor proteins.

Affinity constants (R50%) for receptor binding of VWF were estimated. In addition, dissociation rate constants (off-rates) were calculated by fitting the dissociation phase alone. The binding response representative for a stable protein-ligand interaction (20 seconds after the end of sample injection) was used for calculation. Lower affinities of human monomeric VWF for the mouse receptor proteins MGL1 and MGL2 were estimated, in comparison with VWF-binding to human CLEC10A.

**Table 1:**

| **Immobilized receptor protein** | **Purified VWF monomers as analyte in the mobile phase** | |
|---|---|---|
| | **R_{50%} [µM]** | **Off-rate [10⁻⁴ s⁻¹]** |
| **CLEC10A** | 1.47 | 5.10 |
| **MGL1** | 3.43 | 4.06 |
| **MGL2** | 3.00 | 3.81 |

### Example 3: Inhibition of VWF-binding to MGL2 in the Presence of a Neutralizing Anti-MGL1/2 Antibody

### Materials & Methods

The inhibiting effect of a polyclonal goat anti-MGL1/2 antibody (Prod. No. AF4297, R&D Systems, Wiesbaden, Germany) on VWF binding was investigated by SPR analysis. Lyophilized antibodies were dissolved in running buffer to a concentration of 200 µg/mL. MGL1 and MGL2 were immobilized on a Series S Sensor Chip CM3, respectively. Immobilization of receptor proteins was performed by amine coupling as described before. A surface density of 6,000(±500) RU was targeted. Running buffer and the anti-MGL1/2 antibody were injected for 12 min, respectively, followed by a dual injection of monomeric VWF (2 µM) for 5 min and a final dissociation phase of 8 min. SPR analysis was performed at a flow rate of 20 µL/min at +25°C.

### Results

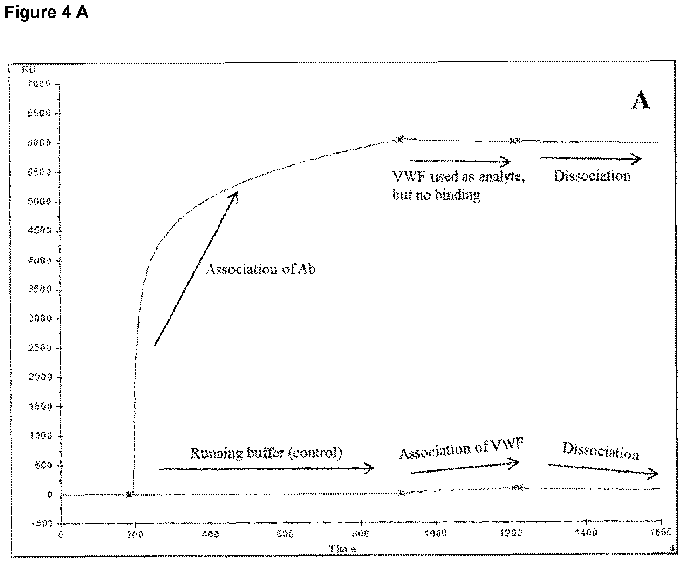

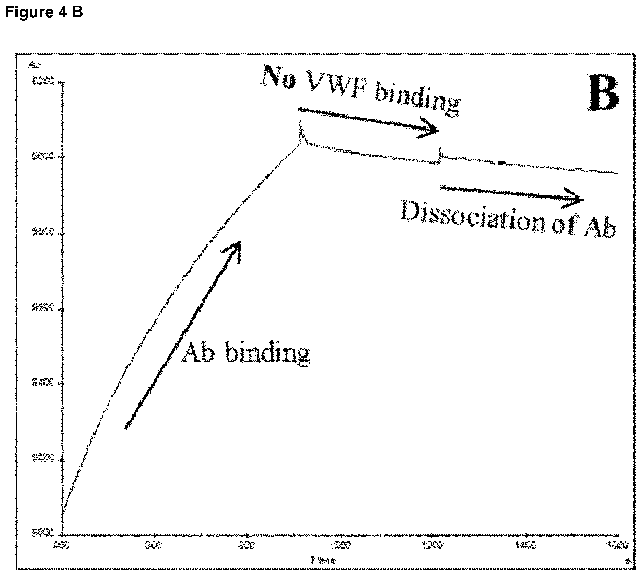

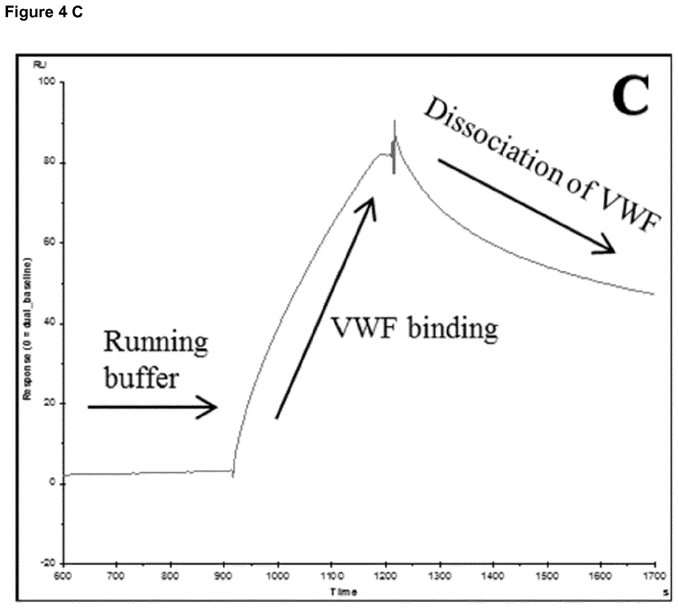

For example (see Figure 4), the neutralizing anti-MGL1/2 showed a strong binding to immobilized MGL2 (see Figures 4A and 4B), resulting in a mass increase. VWF used as analyte did not bind to the immobilized receptor protein as the neutralizing antibody blocked the respective binding domain of the receptor. Consequently, VWF-binding could not be detected. In contrast, VWF strongly bound to immobilized MGL2 in the absence of the neutralizing antibody as demonstrated by the control sample using running buffer (see Figure 4A and 4C).

In conclusion, the receptor-blocking effect of the polyclonal antibody was clearly verified by SPR analysis. As result, analysis by SPR revealed that the antibody completely blocked the interaction of VWF with immobilized MGL1 and MGL2, respectively. Consequently, both antibodies were qualitatively assessed as being applicable to specifically block MGL1 and MGL2.

### Example 4: An Inhibitory Antibody was used to Specifically Block the Mouse Orthologous Receptor Proteins of Human CLEC10A in vivo, Resulting in a Reduced in Vivo Clearance of VWF in Mice

The two CLEC10A orthologous receptor proteins MGL1 and MGL2 exist in the mouse. A polyclonal goat anti-MGL1/2 antibody (Prod. No. AF4297, R&D Systems, Wiesbaden, Germany) was applied for receptor blocking *in vivo.* Moreover, a nonspecific antibody (Prod. No. I5256, Sigma-Aldrich, St. Louis, USA) purified from pooled normal goat serum was used as control treatment.

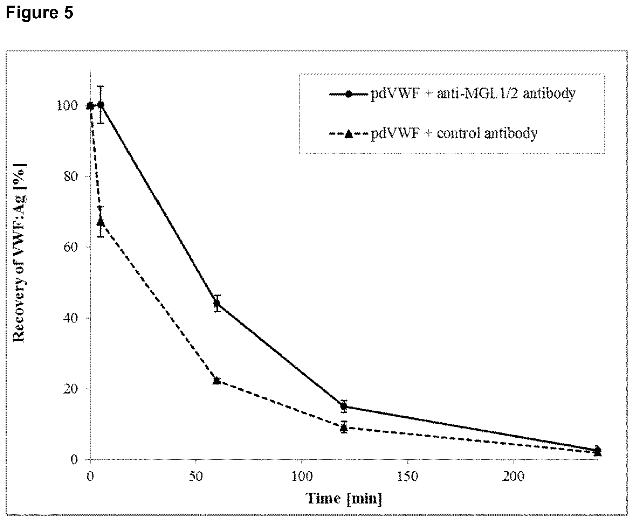

VWF-deficient mice intravenously received 8 mg of the specific inhibiting antibody per kg b.w. to study the effect of MGL1 and MGL2 receptors on VWF clearance *in vivo.* Previously, the lyophilized antibody was dissolved in isotonic NaCl solution (application volume of 5 mL/kg b.w.). The nonspecific antibody was used as control treatment. After 10 minutes, the mice received human pdVWF (200 IU/kg b.w.) as a single intravenous injection (application volume of 5 mL/kg b.w.). The study design included 2 groups of 2 mice each. Blood samples were collected after the administration of VWF (group 1: sampling at 5 and 120 minutes; group 2: sampling at 60 and 240 minutes), the samples were processed to plasma samples and then analyzed by VWF:Ag ELISA. The resulting data are displayed in Figure 5. An overview of the statistical analysis is given in Table 2.

Analysis of PK data revealed that the anti-MGL1/2 antibody treatment of VWF-deficient mice revealed an inhibiting effect on the clearance of human VWF, when compared with the group receiving the control antibody. In the presence of the inhibitory anti-MGL1/2 antibody, the AUC was approximately 1.7-fold higher in comparison to the control treatment, and the plasma clearance rate of VWF was approximately 1.7-fold lower. In conclusion, MGL1 and MGL2 were found to play an important effect in VWF clearance *in vivo* and might be an essential mediator of the uptake of VWF.

In summary, an inhibitory antibody was used to specifically block the carbohydrate recognition domains of the mouse orthologous receptor proteins of human CLEC10A *in vivo,* in order to further evaluate the involvement of the respective receptor proteins in VWF clearance. Analysis of PK data revealed that the anti-MGL1/2 antibody treatment of VWF-deficient mice revealed an inhibiting effect on the clearance of human VWF, when compared with the group receiving the non-specific control antibody. MGL1/MGL2-directed antibodies inhibited degradation of human VWF to a significant extent, indicating that MGL1/MGL2 contributes to binding of VWF and that specific receptor-blocking prevented uptake of VWF *in vivo.* These data suggest that VWF is endocytosed via a receptor-mediated mechanism, and confirm the involvement of human CLEC10A in the uptake of VWF.

**Table 2: Statistical analysis of the in vivo clearance of human VWF in VWF-deficient mice in the presence of an antibody neutralizing the receptor function of both MGL1 and MGL2**

| To assess VWF clearance in the presence of the anti-MGL1/2 antibody, PK data were calculated. In the presence of the inhibitory anti-MGL1/2 antibody, the clearance of VWF was decreased. | | | | | |
|---|---|---|---|---|---|
| **Treatment** | ***C*ₘₐₓ** | ***In vivo* recovery** | **AUC ₀₋₂₄₀ₘᵢₙ** | **Relative AUC** | **Plasma clearance** |
| | [IU/mL] | [%] | [IU*h/mL] | **value*** | [mL/kg/h] |
| pdVWF + control antibody | 3.03 | 61 | 3.03 | 1.0* | 66 |
| pdVWF + anti-MGL1/2 antibody | 4.47 | 89 | 5.14 | 1.7 | 39 |

| | | | | | |
|---|---|---|---|---|---|
| * For the calculation, the AUC of the control treatment with isotonic NaCl solution was defined as 100%, and therefore resulting in factor 1.0. | | | | | |

### Example 5: Generation of Blocking Antibodies to Human CLEC10A

To one skilled in the art there are a number of antibody generation methods that could be used in the discovery of blocking antibodies to human recombinant or membrane-associated CLEC10A. In this example we use antibody phage-display technologies with recombinant CLEC10A for antibody generation and preliminary functional screening. Confirmation of antibody blocking activity is undertaken using cell-based internalisation assays or in vivo pharmacokinetic studies in appropriate models.

A human Fab-based phage display library (Dyax Corp. Cambridge, MA) is used to screen against biotinylated human CLEC10A using methods described previously (WO2013014092 A1). Following three rounds of panning, multiple individual phage clones are selected from each panning round and screening for specific binding to human CLEC10A using Fab-phage ELISA. For any CLEC10A specific phage clones, the Fab region is amplified using PCR and the variable region sequences (heavy and light chain) determined by nucleotide sequencing.

For further functional evaluation, CLEC10A specific Fab antibodies are re-engineered into intact human IgG4 antibodies and expressed using a mammalian expression system as previously described (WO2013014092 A1). Specific binding of these IgG antibodies to CLEC10A is confirmed by ELISA. A panel of unique IgG antibodies with binding specificity for human CLEC10A are identified.

### Screening CLEC10A specific antibodies for function blocking activity

Function blocking activity of the CLEC10A-specific IgG antibodies is assessed by their ability to inhibit the binding of biotinylated *β*GalNAcPAA or vWF to CLEC10A by ELISA. Antibodies showing blocking activity in this assay are then further characterised for their ability to modulate internalisation of fluorophore-conjugated VWF by activated macrophages using flow cytometry.

As any function blocking antibodies identified from this example are fully human in nature, they are readily amenable for therapeutic use in humans.

## Claims

1. An antibody capable of binding to the human receptor protein CLEC10A for use in the treatment of a blood coagulation disorder, wherein said antibody is capable of inhibiting the binding of von Willebrand factor to CLEC10A and said blood coagulation disorder is hemophilia A or von Willebrand disease.

2. The antibody for use according to claim 1, wherein said antibody binds specifically to CLEC10A.

3. The antibody for use according to claim 1 or 2, wherein said antibody is a monoclonal antibody.

4. The antibody for use according to any one of the preceding claims, wherein said CLEC10A has the amino acid sequence shown in SEQ ID NO:1.

5. The antibody for use according to any one of the preceding claims, wherein the half-life of von Willebrand factor is increased by the treatment.

6. The antibody for use according to any one of the preceding claims, wherein the half-life of Factor VIII is increased by the treatment.

7. The antibody for use according to any one of the preceding claims, wherein said treatment further comprises administering a polypeptide selected from the group consisting of Factor VIII, von Willebrand factor and combinations thereof.

8. The antibody for use according to claim 7, wherein said antibody and said polypeptide are administered separately.

9. A pharmaceutical kit comprising (i) an antibody as defined in any one of claims 1 to 4 and (ii) a polypeptide selected from the group consisting of Factor VIII, von Willebrand factor and combinations thereof.

10. The pharmaceutical kit of claim 9, wherein said antibody and said polypeptide are contained in separate compositions.

11. A pharmaceutical kit comprising (i) an antibody as defined in any one of claims 1 to 4 and (ii) a polypeptide selected from the group consisting of Factor VIII, von Willebrand factor and combinations thereof, for simultaneous, separate or sequential use in the treatment of a blood coagulation disorder.

12. The antibody for use according to any one of claims 1 to 4 for use in reducing the clearance of von Willebrand Factor.

13. The antibody for use according to any one of claims 1 to 4 for increasing the half-life of Factor VIII.

## Patentansprüche

1. Antikörper, der in der Lage ist, an das menschliche Rezeptorprotein CLEC10A zu binden, zur Verwendung bei der Behandlung einer Blutgerinnungsstörung, wobei der Antikörper in der Lage ist, die Bindung des Von-Willebrand-Faktors an CLEC10A zu hemmen und es sich bei der Blutgerinnungsstörung um Hämophilie A oder Von-Willebrand-Syndrom handelt.

2. Antikörper zur Verwendung nach Anspruch 1, wobei der Antikörper spezifisch an CLEC10A bindet.

3. Antikörper zur Verwendung nach Anspruch 1 oder 2, wobei der Antikörper ein monoklonaler Antikörper ist.

4. Antikörper zur Verwendung nach einem der vorhergehenden Ansprüche, wobei das CLEC10A die in SEQ ID NO:1 gezeigte Aminosäuresequenz aufweist.

5. Antikörper zur Verwendung nach einem der vorhergehenden Ansprüche, wobei durch die Behandlung die Halbwertszeit des Von-Willebrand-Faktors erhöht wird.

6. Antikörper zur Verwendung nach einem der vorhergehenden Ansprüche, wobei durch die Behandlung die Halbwertszeit von Faktor VIII erhöht wird.

7. Antikörper zur Verwendung nach einem der vorhergehenden Ansprüche, wobei die Behandlung ferner die Verabreichung eines Polypeptids umfasst, das aus der Gruppe ausgewählt ist, die aus Faktor VIII, Von-Willebrand-Faktor und Kombinationen davon besteht.

8. Antikörper zur Verwendung nach Anspruch 7, wobei der Antikörper und das Polypeptid getrennt verabreicht werden.

9. Pharmazeutisches Kit, umfassend (i) einen wie in einem der Ansprüche 1 bis 4 definierten Antikörper und (ii) ein Polypeptid, das aus der Gruppe ausgewählt ist, die aus Faktor VIII, Von-Willebrand-Faktor und Kombinationen davon besteht.

10. Pharmazeutisches Kit nach Anspruch 9, wobei der Antikörper und das Polypeptid in getrennten Zusammensetzungen enthalten sind.

11. Pharmazeutisches Kit, umfassend (i) einen wie in einem der Ansprüche 1 bis 4 definierten Antikörper und (ii) ein Polypeptid, das aus der Gruppe ausgewählt ist, die aus Faktor VIII, Von-Willebrand-Faktor und Kombinationen davon besteht, zur gleichzeitigen, getrennten oder aufeinanderfolgenden Verwendung bei der Behandlung einer Blutgerinnungsstörung.

12. Antikörper zur Verwendung nach einem der Ansprüche 1 bis 4 zur Verwendung bei der Verringerung der Clearance des Von-Willebrand-Faktors.

13. Antikörper zur Verwendung nach einem der Ansprüche 1 bis 4 zur Erhöhung der Halbwertszeit von Faktor VIII.

## Revendications

1. Anticorps capable de se lier à la protéine CLEC10A réceptrice humaine destiné à être utilisé dans le traitement d'un trouble de la coagulation du sang, ledit anticorps étant capable d'inhiber la liaison du facteur de von Willebrand à la CLEC10A et ledit trouble de la coagulation du sang est l'hémophilie A ou la maladie de von Willebrand.

2. Anticorps destiné à être utilisé selon la revendication 1, ledit anticorps se liant spécifiquement à la CLEC10A.

3. Anticorps destiné à être utilisé selon les revendications 1 ou 2, ledit anticorps étant un anticorps monoclonal.

4. Anticorps destiné à être utilisé selon l'une quelconque des revendications précédentes, dans lequel ladite CLEC10A a la séquence d'acides aminés présentée dans la SEQ ID NO: 1.

5. Anticorps destiné à être utilisé selon l'une quelconque des revendications précédentes, dans lequel la demi-vie du facteur de von Willebrand est accrue par le traitement.

6. Anticorps destiné à être utilisé selon l'une quelconque des revendications précédentes, dans lequel la demi-vie du facteur VIII est accrue par le traitement.

7. Anticorps destiné à être utilisé selon l'une quelconque des revendications précédentes, dans lequel ledit traitement comprend en outre l'administration d'un polypeptide choisi dans le groupe constitué par le facteur VIII, le facteur de von Willebrand et des combinaisons de ceux-ci.

8. Anticorps destiné à être utilisé selon la revendication 7, ledit anticorps et ledit polypeptide étant administrés séparément.

9. Trousse pharmaceutique comprenant (i) un anticorps, tel que défini dans l'une quelconque des revendications 1 à 4, et (ii) un polypeptide choisi dans le groupe constitué par le facteur VIII, le facteur de von Willebrand et des combinaisons de ceux-ci.

10. Trousse pharmaceutique de la revendication 9, dans laquelle ledit anticorps et ledit polypeptide sont contenus dans des compositions séparées.

11. Trousse pharmaceutique comprenant (i) un anticorps, tel que défini dans l'une quelconque des revendications 1 à 4, et (ii) un polypeptide choisi dans le groupe constitué par le facteur VIII, le facteur de von Willebrand et des combinaisons de ceux-ci destinés à une utilisation simultanée, séparée ou séquentielle dans le traitement d'un trouble de la coagulation du sang.

12. Anticorps destiné à être utilisé selon l'une quelconque des revendications 1 à 4, pour une utilisation dans la réduction de la clairance du facteur de von Willebrand.

13. Anticorps destiné à être utilisé selon l'une quelconque des revendications 1 à 4, pour augmenter la demi-vie du facteur VIII.
